(19) Europäisches Patentamt
European Patent Office
Office européen des brevets

(11) Numéro de publication : **0 416 962 B1**

# (12) FASCICULE DE BREVET EUROPEEN

(45) Date de publication du fascicule du brevet : **22.09.93 Bulletin 93/38**

(51) Int. Cl.$^5$ : **A61K 7/13**

(21) Numéro de dépôt : **90402062.5**

(22) Date de dépôt : **18.07.90**

(54) **Procédé de coloration des fibres kératiniques avec des colorants indoliques et des précurseurs de colorants d'oxydation et agents de teinture.**

(30) Priorité : **21.07.89 FR 8909836**

(43) Date de publication de la demande : **13.03.91 Bulletin 91/11**

(45) Mention de la délivrance du brevet : **22.09.93 Bulletin 93/38**

(84) Etats contractants désignés : **AT BE CH DE DK ES FR GB GR IT LI NL SE**

(56) Documents cités :
**EP-A- 0 350 385**
**FR-A- 2 613 221**
**GB-A- 2 207 443**

(73) Titulaire : **L'OREAL**
**14, Rue Royale**
**F-75008 Paris (FR)**

(72) Inventeur : **Vayssie, Charles**
**28, Chemin de Savigny**
**F-93420 Villepinte (FR)**
Inventeur : **Bauer, Daniel**
**8bis Allée La Fontaine**
**F-93340 Le Raincy (FR)**
Inventeur : **Richard, Françoise**
**27, rue Clotilde Gaillard**
**F-93100 Montreuil (FR)**
Inventeur : **Junino, Alex**
**16, Allée Docteur Bergonié**
**F-93190 Livry-Gargan (FR)**

(74) Mandataire : **Casalonga, Axel et al**
**BUREAU D.A. CASALONGA - JOSSE**
**Morassistrasse 8**
**D-80469 München (DE)**

EP 0 416 962 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à un nouveau procédé de coloration des fibres kératiniques, plus particulièrement des fibres kératiniques humaines, telles que les cheveux, avec un colorant indolique et un précurseur de colorant d'oxydation.

Les couleurs des cheveux teints avec des compositions contenant le 5,6-dihydroxyindole diffèrent suivant la couleur d'origine, la nature des cheveux et le mode d'application. Le résultat obtenu peut être jugé trop clair ou trop terne. Il est donc intéressant de pouvoir nuancer cette coloration, par exemple par application d'une seconde composition, soit immédiatement après la première application, soit après un certain temps pouvant aller jusqu'à plusieurs jours. Il est également souhaitable de pouvoir procéder à ce nuançage, soit localement, soit sur l'ensemble de la chevelure.

Les inventeurs ont constaté, d'une façon surprenante, qu'en procédant à l'application, dans un premier temps, d'une composition contenant un colorant indolique sans utiliser d'agent oxydant autre que l'air, et dans un second temps, d'une composition contenant un précurseur de colorant d'oxydation du type para, il y avait un approfondissement de la couleur et non pas une simple juxtaposition des couleurs obtenues avec lesdits colorants pris individuellement.

Par ailleurs, les inventeurs ont également constaté que les colorations ainsi obtenues étaient beaucoup plus chaudes que les colorations individuelles obtenues, soit avec le colorant indolique seul, soit avec les précurseurs de colorant d'oxydation seuls.

Ce procédé ne fait pas appel aux catalyseurs d'oxydation utilisés en pré ou post-traitement. En-dehors de l'oxygène de l'air, on peut éventuellement utiliser dans le second temps, de l'eau oxygénée.

L'invention a donc pour objet un nouveau procédé de teinture mettant en oeuvre, dans une première étape, un colorant indolique, et dans une seconde étape, un précurseur de colorant d'oxydation de type para.

D'autres objets de l'invention apparaîtront à la lecture de la description et des exemples qui suivent.

Le procédé conforme à l'invention destiné à la teinture des fibres kératiniques et en particulier des fibres kératiniques humaines, telles que les cheveux, est caractérisé par le fait que l'on applique sur ces fibres :

- dans une première étape, une composition (A) contenant dans un milieu approprié pour la teinture et exempt de tout agent oxydant, au moins un colorant indolique répondant à la formule :

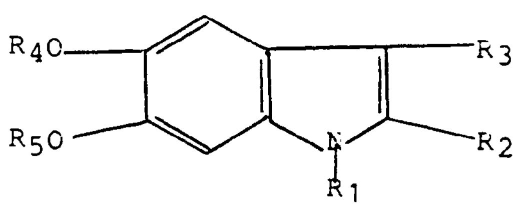

(I)

dans laquelle :

$R_1$ représente un atome d'hydrogène, un groupement alkyle inférieur ou un groupement $-SiR_6R_7R_8$;

$R_2$ et $R_3$, identiques ou différents, représentent un atome d'hydrogène ou bien un groupement alkyle inférieur, un groupement carboxyle, un groupement (alcoxy inférieur)carbonyle ou un groupement $-COOSiR_6R_7R_8$;

$R_4$ et $R_5$, identiques ou différents, représentent un atome d'hydrogène, un groupement alkyle, linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement $-SiR_6R_7R_8$, un groupement $-P(O)(OR_9)_2$, un groupement $-SO_2OR_9$ ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :

$>P(C)OR_9$, ou bien $>CR_{10}R_{11}$;

$R_9$ et $R_{10}$ représentent un atome d'hydrogène ou un groupement alkyle inférieur, $R_{11}$ représentant un groupement alcoxy inférieur ou un groupement mono- ou dialkylamino, $R_6$, $R_7$ et $R_8$, identiques ou différents, représentant des groupements alkyle inférieurs, linéaires ou ramifiés;

et les sels d'addition avec des acides minéraux ou organiques, ainsi que les sels de métaux alcalins, alcalino-terreux ou d'amines correspondants; et

- dans une deuxième étape, on applique une composition (B) contenant dans un milieu approprié pour la teinture, au moins un précurseur de colorant d'oxydation para, choisi parmi les paraphénylènediamines, les paraaminophénols, les bases para hétérocycliques dérivées de la pyridine ou de la pyrimidine et/ou

au moins un colorant d'oxydation "rapide" choisi parmi les dérivés trihydroxylés du benzène, les diamino hydroxybenzènes, les amino dihydroxybenzènes, les aminohydroxybenzènes substitués, les triaminobenzènes, les 1,2-dihydroxybenzènes substitués, la braziline, l'hematoxyline et l'extrait d'orcanete.

Cette deuxième composition est éventuellement mélangée au moment de l'emploi avec une solution de peroxyde d'hydrogène.

Conformément à l'invention, le premier temps et le deuxième temps du procédé peuvent être séparés par un rinçage. Lorsque les fibres traitées sont rincées après l'application de la composition (A), il est possible éventuellement de sécher les cheveux avant l'application de la deuxième composition contenant les précurseurs de colorant d'oxydation.

Les colorants indoliques préférés répondant à la formule (I), sont choisis parmi le 5,6-dihydroxy indole, le 2-méthyl 5,6-dihydroxyindole, le 5-méthoxy 6-hydroxyindole, le (5 ou 6)-acétoxy(6- ou 5-) hydroxyindole, le 2-carboxy 5,6-dihydroxyindole, le 3-méthyl 5,6-dihydroxyindole, le 2,3-diméthyl 5,6-dihydroxyindole et leurs sels.

Le 5,6-dihydroxyindole est particulièrement préféré; il est utilisé tel quel ou sous l'une de ses formes protégées telles que par exemple le diacétate.

Les précurseurs de colorant para sont choisis parmi les composés répondant à la formule :

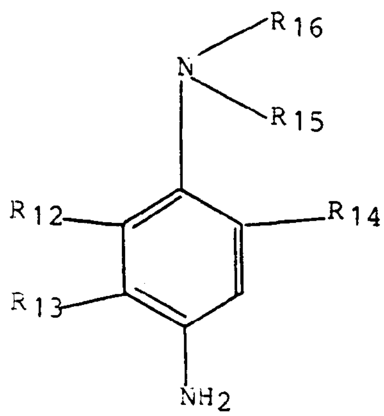

(II)

dans laquelle:

$R_{12}$,$R_{13}$,$R_{14}$, identiques ou différents, désignent un atome d'hydrogène ou d'halogène, un radical alkyle inférieur, un radical alcoxy inférieur;

$R_{15}$ et $R_{16}$, identiques ou différents, désignent un atome d'hydrogène, un groupement alkyle inférieur, hydroxyalkyle inférieur, alcoxyalkyle inférieur, carbamylalkyle inférieur, sulfoalkyle inférieur, mésylaminoalkyle inférieur, acétylaminoalkyle inférieur, uréidoalkyle inférieur, carbéthoxyaminoalkyle inférieur, pipéridinoalkyle inférieur, morpholinoalkyle inférieur, ou bien $R_{15}$ et $R_{16}$ forment conjointement avec l'atome d'azote auquel ils sont liés, un hétérocycle pipéridino, morpholino, sous réserve que $R_{12}$ et $R_{14}$ représentent un atome d'hydrogène lorsque $R_{15}$ et $R_{16}$ ne représentent pas un atome d'hydrogène;

ainsi que leurs sels d'addition d'acide, tels que chlorhydrates, bromhydrates et sulfates.

Dans les formules précitées, un radical alkyle ou alcoxy inférieur désigne un radical ayant 1 à 6 atomes de carbone.

Parmi les composés de formule (II) particulièrement préférés, on peut citer la p-phénylène diamine, la p-toluylènediamine, la méthoxyparaphénylène diamine, la chloroparaphénylènediamine, la 2,6-diméthyl-p-phénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthyl paraphénylènediamine, la 3-méthyl 4-amino N,N-diéthylaniline, la N,N-di(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-β-méthoxyéthylaniline, la 3-méthyl 4-amino N,N-(éthyl, β-acétylaminoéthyl)aniline, la 4-amino N,N-(éthyl,β-mésylaminoéthyl)aniline, la 3-méthyl 4-amino N,N-(éthyl, β-mésylaminoéthyl)aniline, la 4-amino N,N-(éthyl,β-sulfoéthyl)aniline, la 3-méthyl 4-amino N,N-(éthyl,β-sulfoéthyl)aniline, la N-(4-aminophényl)morpholine, la N-(4-aminophényl)pipéridine, la 2,3-diméthyl-p-phénylènediamine, l'isopropyl-p-phénylènediamine, et leurs sels.

Parmi les paraaminophénols, on peut citer le paraaminophénol, le N-méthyl-p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol.

Les précurseurs de colorants d'oxydation para hétérocycliques sont dérivés de la pyridine, tels que la 2,5-diaminopyridine, la 2-hydroxy 5-aminopyridine et ceux dérivés de la pyrimidine sont en particulier constitués par la tétraaminopyrimidine.

Les colorants d'oxydation dits "rapides" sont choisis parmi le 1,2,4-trihydroxybenzène, le 1,2,4-trihydroxy 5-alkyl($C_1$-$C_6$)benzène, la 4-amino résorcine, le 1,2,3-trihydroxybenzène, le 4-méthyl 1,2-dihydroxybenzène, le 2-amino 1,4-dihydroxybenzène, le 2-amino 4-méthoxyphénol, le 2,4-diaminophénol, le 3-méthoxy 1,2-dihydroxybenzène, le 1,4-dihydroxy 2N,N-diéthylaminobenzène, le 2,5-diamino 4-méthoxy 1-hydroxybenzène, le 4,6-diméthoxy 3-amino 1-hydroxybenzène, le 2,6-diméthyl 4N-p-hydroxyphénylamino 1-hydroxybenzène, le 1,5-diamino 2-méthyl 4N-p-hydroxyphénylaminobenzène, et leurs sels.

La composition (B) peut contenir en outre un coupleur choisi parmi les phénols, les diphénols, les m-phénylènediamines, les m-aminophénols, les hydroxynaphtalènes et les coupleurs hétérocycliques dans des proportions n'excédant pas 3% en poids par rapport au poids total de la composition.

Le colorant indolique est utilisé dans la composition (A) conforme à l'invention, dans des proportions comprises entre 0,1 et 5% en poids et de préférence entre 0,2 et 2% en poids, par rapport au poids total de la composition.

Les précurseurs de colorants de type para ou les colorants d'oxydation "rapides" sont utilisés dans la composition (B) conforme à l'invention dans des proportions comprises entre 0,05 et 3% en poids et en particulier entre 0,1 et 2% en poids par rapport au poids total de la composition.

Le pH de la composition (A) contenant le colorant indolique est compris entre 4 et 11 et de préférence entre 6 et 10.

Le pH de la composition (B) contenant le précurseur de colorant de type para et/ou le colorant d'oxydation "rapide" est compris entre 7 et 12 et de préférence entre 8 et 11.

Les pH sont ajustés à la valeur désirée à l'aide d'agents alcalinisants ou acidifiants, connus en eux-mêmes et cosmétiquement acceptables lorsque les compositions sont destinées à être appliquées sur les cheveux humains.

Ces compositions peuvent adopter des formes diverses, telles que les lotions plus ou moins épaissies, les gels; elles peuvent être conditionnées en aérosol, en particulier pour être distribuées sous forme de mousse.

Le milieu aqueux peut renfermer, en outre, des solvants présents généralement dans des proportions pouvant aller jusqu'à 30% par rapport au poids total de la composition et choisis parmi les alcools inférieurs, tels que l'alcool éthylique, l'alcool propylique ou isopropylique, l'alcool tertiobutylique; des glycols, tels que l'éthylèneglycol, le propylèneglycol; des éthers de glycols, tels que les éthers monométhylique, monoéthylique et monobutylique de l'éthylèneglycol, l'acétate du monoéthyléther de l'éthylèneglycol, le monométhyléther du propylèneglycol et du dipropylène glycol, le lactate de méthyle.

Les solvants particulièrement préférés sont constitués par l'alcool éthylique et le propylèneglycol.

Ces compositions peuvent également contenir des adjuvants bien connus en coloration capillaire, tels que les agents antioxydants, des agents épaississants, des agents de conditionnement; elles peuvent également renfermer des agents tensio-actifs, ainsi que des silicones, notamment des silicones volatiles.

Les agents épaississants sont choisis parmi l'alginate de sodium, la gomme arabique, la gomme de guar ou de caroube, les hétérobiopolysaccharides, tels que la gomme de xanthane, les dérivés de cellulose, tels que la méthylcellulose, l'hydroxyéthylcellulose, l'hydroxyméthylcellulose, l'hydroxypropylcellulose, la carboxyméthylcellulose et des polymères divers ayant des fonctions épaississantes, tels que les dérivés d'acide acrylique. On peut également utiliser des agents épaississants minéraux, tels que la bentonite.

Ces agents épaississants sont présents de préférence dans des proportions comprises entre 0,1 et 5% en poids et en particulier entre 0,5 et 3% en poids par rapport au poids total de la composition.

Le procédé est mis en oeuvre en appliquant sur les fibres dans un premier temps, la composition (A) et en la maintenant au contact des fibres pendant un temps de pose de 1 à 60 minutes, de préférence de 2 à 30 minutes, éventuellement suivi d'un rinçage, d'un lavage et d'un séchage. Cette application peut être répétée.

Elle est suivie par l'application de la composition (B), qui est maintenue au contact des fibres pendant une durée de 1 à 60 minutes, de préférence de 1 à 20 minutes. Les fibres sont alors rincées, lavées éventuellement au shampooing et séchées.

L'application de la composition (B) peut se faire immédiatement ou après un laps de temps variant de quelques minutes à plusieurs jours.

L'invention a également pour objet un agent de coloration des fibres kératiniques, en particulier des fibres kératiniques humaines, telles que les cheveux, caractérisé par le fait qu'il comprend deux composants, le premier composant étant constitué par une composition (A) contenant dans un milieu approprié pour la teinture, au moins un colorant indolique de formule (I) telle que définie ci-dessus et le second composant étant constitué par une composition (B) contenant dans un milieu approprié pour la teinture, au moins un précurseur de colorant d'oxydation de type para et/ou un colorant d'oxydation "rapide" tel que défini ci-dessus.

Cet agent de teinture peut être stocké dans un dispositif à compartiments multiples ou kit de teinture, les différents composants étant stockés dans des compartiments séparés.

Les exemples suivants sont destinées à illustrer l'invention.

EXEMPLE 1

On prépare les compositions ($A_1$) et ($B_1$) suivantes :

Composition ($A_1$) :

- 5,6-dihydroxyindole 1,0 g
- Ethanol 10,0 g
- Lauryléthersulfate de sodium 1,0 g
- pH = 8,5
- Eau qsp 100,0 g

Cette composition est appliquée pendant 10 minutes sur des cheveux gris naturel à 90% de blancs, à température ambiante. Les cheveux sont alors lavés au shampooing, puis rincés et séchés. Ils sont colorés en blond foncé.

Composition ($B_1$) :

- Paraaminophenol 1,0 g
- Ethanol 12,0 g
- Hydroxyéthylcellulose vendue sous la dénomination de NATROSOL 250 HHR par la Société AQUALON 1,5 g
- Soude qs pH = 9,5
- Eau qsp 100,0 g

Au moment de l'emploi on ajoute un volume égal d'eau oxygénée à 6%. Cette composition est appliquée sur les cheveux prétraités par ($A_1$) pendant 3 minutes à température ambiante. Les cheveux sont alors rincés et séchés. Ils sont colorés en châtain acajou.

EXEMPLE 2

On applique dans les mêmes conditions la composition ($A_1$) décrite dans l'exemple 1, puis on applique la composition ($B_2$) suivante :

- N,N-di-(β-hydroxyéthyl)paraphénylènne diamine 1,0 g
- Ether monobutylique de l'éthylène glycol 15,0 g
- Lauryléthersulfate de sodium à 2 moles d'oxyde d'éthylène, vendu sous la dénomination SACTIPON 85033 par la Société LEVER 3,5 g
- Soude qs pH = 9,5
- Eau qsp 100,0 g

Cette composition est mélangée au moment de l'emploi avec un volume égal d'eau oxygénée à 6%. Après trois minutes de pose, les cheveux sont rincés et séchés; ils sont colorés en châtain.

EXEMPLE 3

On opère comme dans l'exemple 1 en utilisant une composition ($B_3$) correspondant à ($B_1$) mais qui contient 1% de N-méthylparaaminophénol à la place du paraaminophénol. Les cheveux sont colorés en châtain cuivré acajou.

EXEMPLE 4

On opère comme dans l'exemple 2 en utilisant une composition ($B_4$) qui contient du 2-méthyl 4-amino phénol à la place de la N,N-di-(β-hydroxyéthyl)para phénylènediamine. On obtient une couleur châtain clair doré cuivré.

EXEMPLE 5

On opère comme dans l'exemple 1 en utilisant une composition ($B_5$) contenant 1% de paraphénylène diamine à la place du paraaminophénol. On obtient une couleur châtain foncé.

EXEMPLE 6

On opère comme dans l'exemple 5, sauf que l'on utilise 0,12% de paraphénylènediamine. On obtient une couleur châtain clair doré.

EXEMPLE 7

On applique dans les mêmes conditions la composition ($A_1$) décrite dans l'exemple 1, puis on applique la composition ($B_7$) suivante :

- Paraphénylènediamine 1,0 g
- Ethanol 12,0 g
- Hydroxyéthylcellulose 1,5 g
- Soude qs pH = 9,5
- Eau qsp 100,0 g

Cette composition est appliquée sur les cheveux teints avec la composition ($A_1$). On laisse poser pendant 5 minutes, puis on rince les cheveux et on les sèche, ceux-ci sont teints en une couleur blond foncé doré.

EXEMPLE 8

Des mèches grises, 90% de blancs, sont teintes avec la composition ($A_1$) contenant le 5,6-dihydroxy indole dans les conditions précédemment décrites. On obtient ainsi après séchage une mèche colorée en blond foncé.

On applique sur cette mèche pendant 3 minutes, une composition ($B_8$) contenant 1% en poids de 2,5-diaminopyridine dans l'eau à laquelle on a rajouté un poids égal d'une solution d'eau oxygénée à 6% (pH du mélange : 7,3). Après rinçage et séchage, la mèche a une teinte plus soutenue légèrement acajou irisé.

EXEMPLE 9

Par application sur des mèches de cheveux gris d'une solution à 0,5% de 2-méthyl 5,6-dihydroxyindole à pH 10, on obtient après 10 minutes de pose puis rinçage et séchage, des mèches bond clair cendré.

Si dans un deuxième temps on applique une solution ($B_9$) similaire à la composition ($B_1$) mais contenant 2% de para-aminophénol, on obtient une mèche teinte en blond doré à reflets rouges.

EXEMPLE 10

On traite des mèches de cheveux gris à 90% de blancs par une solution à 0,5% de 5,6-dihydroxyindole à pH 9 pendant 15 minutes. Après rinçage et séchage, on obtient des mèches teintées en blond métallique.

Si on applique ensuite une solution hydroalcoolique à 0,5% de 1,2,4-trihydroxybenzène en présence d'eau oxygénée à 3% (pH du mélange environ 7) pendant 3 minutes, les mèches obtenues sont blond cendré irisé.

EXEMPLE 11

On prépare une solution hydroalcoolique à 2,5% de 5-méthoxy 6-hydroxyindole que l'on ajuste à pH 9 par de la soude. Cette solution est appliquée pendant 10 minutes sur des cheveux gris à 90% de blancs. Après rinçage et séchage, les cheveux sont teints en gris légèrement cendré.

On applique alors un mélange à parts égales d'une solution à 0,5% de N-β-méthoxyéthylparaphénylène diamine ajustée à pH 10 et d'eau oxygénée à 6% pendant 3 minutes. Après rinçage et séchage, les cheveux sont colorés en châtain clair violine.

EXEMPLE 12

On prépare une solution hydroalcoolique à 30% d'alcool, de 5-hydroxy 6-méthoxyindole à 2,5% que l'on ajuste à pH 9 par de la soude. On ajoute ensuite 1% de lauryléthersulfate de sodium.

A l'aide de cette solution, on traite (rapport de bain 5 g pour 1 g de cheveux) des mèches de cheveux naturels gris à 90% de cheveux blancs pendant 10 minutes à température ambiante. Après rinçage et séchage, on obtient des cheveux gris clair très légèrement cendré.

Sur une mèche précédemment traitée, on applique une solution aqueuse de N-(β-méthoxyéthyl) paraphénylènediamine à 0,5% ajustée à pH 10 par addition de soude que l'on mélange avec une quantité égale d'eau oxygénée à 6%. Le traitement est effectué pendant 3 minutes avec un rapport de bain de 5 g pour 1 g de cheveu. Après rinçage, lavage et séchage, on obtient une mèche gris foncé légèrement bleuté.

EXEMPLE 13

On traite des mèches de cheveux gris à 90% de blancs, par une solution hydroalcoolique à 35% d'alcool, de 5-acétoxy 6-hydroxyindole à 1% en présence de 1% de lauryléthersulfate de sodium. L'application est effectuée à raison de 5 g par g de cheveux pendant 10 minutes.

Après rinçage et séchage, on obtient des cheveux blond clair cendré.

Dans une deuxième étape, on applique aux cheveux ainsi traités un mélange à parts égales d'une solution à 1% de N,N-bis(β-hydroxyéthyl)-paraphénylène diamine à pH 10 et d'eau oxygénée à 6 volumes pendant 3 minutes à raison de 5 g de mélange par g de cheveux.

Après rinçage, lavage par un shampooing et séchage, on obtient des cheveux gris foncé à reflets violine.

## Revendications

1. Procédé de coloration des fibres kératiniques, en particulier des fibres kératiniques humaines, caractérisé par le fait que l'on applique :
   - dans un premier temps, une composition (A) contenant dans un milieu approprié pour la teinture exempt de tout agent oxydant et de tout catalyseur d'oxydation, au moins un colorant indolique répondant à la formule :

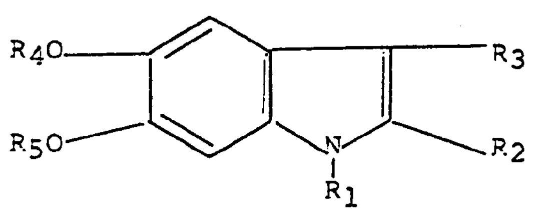

(I)

dans laquelle :

$R_1$ représente un atome d'hydrogène, un groupement alkyle inférieur ou un groupement -$SiR_6R_7R_8$;

$R_2$ et $R_3$, identiques ou différents, représentent un atome d'hydrogène ou bien un groupement alkyle inférieur, un groupement carboxyle, un groupement alcoxy inférieur carbonyle ou un groupement -$COOSiR_6R_7R_8$;

$R_4$ et $R_6$, identiques ou différents, représentent un atome d'hydrogène, un groupement alkyle, linéaire ou ramifié en $C_1$-$C_{20}$, un groupement formyle, un groupement acyle en $C_2$-$C_{20}$, linéaire ou ramifié, un groupement alcénoyle en $C_3$-$C_{20}$, linéaire ou ramifié, un groupement -$SiR_6R_7R_8$, un groupement -$P(O)(OR_9)_2$, un groupement -$SO_2OR_9$ ou bien $R_4$ et $R_5$, conjointement avec les atomes d'oxygène auxquels ils sont rattachés, forment un cycle contenant éventuellement un groupement carbonyle, un groupement méthylène, un groupement thiocarbonyle ou un groupement :

$>P(O)OR_9$, ou bien $>CR_{10}R_{11}$ ;

$R_9$ et $R_{10}$ représentent un atome d'hydrogène ou un groupement alkyle inférieur, $R_{11}$ représentant un groupement alcoxy inférieur ou un groupement mono- ou dialkylamino inférieur, $R_6$, $R_7$ et $R_6$, identiques ou différents, représentant des groupements alkyle inférieurs, linéaires ou ramifiés;

et les sels d'addition avec des acides minéraux ou organiques, ainsi que les sels de métaux alcalins, alcalino-terreux ou d'amines correspondants;

- dans un deuxième temps, on applique sur ces fibres, une composition (B) contenant dans un milieu approprié pour la teinture exempt de tout catalyseur d'oxydation, au moins un précurseur de colorant d'oxydation para, choisi parmi les paraphénylènediamines, les paraaminophénols et les bases para hétérocycliques choisies parmi les dérivés de la pyridine ou de la pyrimidine et/ou au moins un colorant d'oxydation dit "rapide" choisi parmi les dérivés trihydroxylés du benzène, les diaminohydroxybenzènes, les aminodihydroxybenzènes, les aminohydroxybenzènes substitués, les triaminobenzènes, les 1,2-dihydroxybenzènes substitués, la braziline, l'hématoxyline, et l'extrait d'orcanète, la composition (B) étant éventuellement mélangée, au moment de l'emploi, à une solution de peroxyde d'hydrogène.

2. Procédé selon la revendication 1, caractérisé par le fait que l'on procède à un rinçage des fibres traitées après application de la composition (A).

3. Procédé selon la revendication 2, caractérisé par le fait que l'on fait suivre ce rinçage par une étape de séchage des cheveux avant l'application de la composition (B).

4. Procédé selon l'une quelconque des revendications 1 à 3, caractérisé par le fait que les colorants indoliques de formule (I) sont choisis parmi le 5,6-dihydroxyindole, le 2-méthyl 5,6-dihydroxyindole, le 5-méthoxy 6-hydroxyindole, le (5 ou 6)-acétoxy(6- ou 5-)hydroxyindole, le 2-carboxy 5,6-dihydroxyindole, le 3-méthyl 5,6-dihydroxyindole, le 2,3-diméthyl 5,6-dihydroxyindole et leurs sels.

5. Procédé selon l'une quelconque des revendications 1 à 3, caractérisé par le fait que les paraphénylènediamines répondent à la formule (II) :

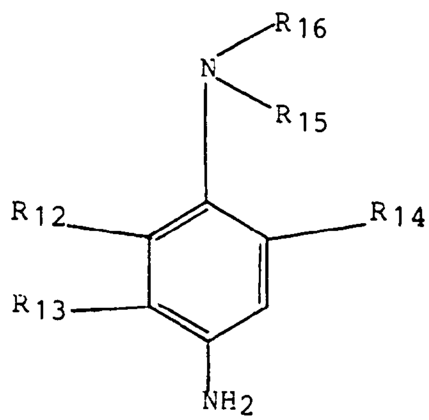

(II)

dans laquelle :

$R_{12}$, $R_{13}$, $R_{14}$, identiques ou différents, désignent un atome d'hydrogène ou d'halogène, un radical alkyle inférieur, un radical alcoxy inférieur;

$R_{15}$ et $R_{16}$, identiques ou différents, désignent un atome d'hydrogène, un groupement alkyle inférieur, hydroxyalkyle inférieur, alcoxyalkyle inférieur, carbamylalkyle inférieur, sulfoalkyle inférieur, mésylaminoalkyle inférieur, acétylaminoalkyle inférieur, uréidoalkyle inférieur, carbéthoxyaminoalkyle inférieur, pipéridinoalkyle inférieur, morpholino alkyle inférieur, ou bien $R_{15}$ et $R_{16}$ forment, conjointement avec l'atome auquel ils sont liés, un hétérocycle pipéridino ou morpholino, sous réserve que $R_{12}$ et $R_{14}$ représentent un atome d'hydrogène lorsque $R_{15}$ et $R_{16}$ ne représentent pas un atome d'hydrogène, ainsi que les sels correspondants.

6. Procédé selon l'une quelconque des revendications 1 à 3 et 5, caractérisé par le fait que les paraphénylènediamines sont choisies parmi la p-phénylènediamine, la p-toluylènediamine, la méthoxyparaphénylènediamine, la chloroparaphénylène diamine, la 2,6-diméthyl-p-phénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthyl paraphénylènediamine, la 3-méthyl 4-amino N,N-diéthylaniline, la N,N-di(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino

N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-β-méthoxyéthylaniline, la 3-méthyl 4-amino N,N-(éthyl, β-acétylaminoéthyl)aniline, la 4-amino N,N-(éthyl,β-mésylaminoéthyl)aniline, la 3-méthyl 4-amino N,N-(éthyl, β-mésylaminoéthyl)aniline, la 4-amino N,N-(éthyl,β-sulfoéthyl)aniline, la 3-méthyl 4-amino N,N-(éthyl,β-sulfoéthyl)aniline, la N-(4-aminophényl)morpholine, la N-(4-aminophényl)pipéridine, la 2,3-diméthyl-p-phénylènediamine, l'isopropyl-p-phénylènediamine, et leurs sels.

7. Procédé selon l'une quelconque des revendications 1 à 3, caractérisé par le fait que les paraaminophénols sont choisis parmi le paraaminophénol, le N-méthyl p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol.

8. Procédé selon l'une quelconque des revendications 1 à 3, caractérisé par le fait que les colorants d'oxydation para hétérocycliques sont choisis parmi la 2,5-diaminopyridine, la 2-hydroxy 5-amino pyridine et la tétraaminopyrimidine.

9. Procédé selon l'une quelconque des revendications 1 à 3, caractérisé par le fait que le colorant d'oxydation "rapide" est choisi parmi le 1,2,4-trihydroxybenzène, le 1,2,4-trihydroxy 5-alkyl ($C_1$-$C_6$)benzène, la 4-amino résorcine, le 1,2,3-trihydroxybenzène, le 4-méthyl 1,2-dihydroxybenzène, le 2-amino 1,4-dihydroxy benzène, le 2-amino 4-méthoxy phénol, le 2,4-diaminophénol, le 3-méthoxy 1,2-dihydroxy benzène, le 1,4-dihydroxy 2N,N-diéthylaminobenzène, le 2,5-diamino 4-méthoxy 1-hydroxybenzène, le 4,6-diméthoxy 3-amino 1-hydroxybenzène, le 2,6-diméthyl 4N-p-hydroxy phénylamino 1-hydroxybenzène, le 1,5-diamino 2-méthyl 4N-p-hydroxyphénylaminobenzène, et leurs sels.

10. Procédé selon l'une quelconque des revendications 1 à 9, caractérisé par le fait que le milieu approprié pour la teinture est un milieu aqueux constitué par de l'eau, un mélange eau/solvant(s).

11. Procédé selon l'une quelconque des revendications 1 à 4 et 10, caractérisé par le fait que le pH de la composition (A) est compris entre 4 et 11, et de préférence entre 6 et 10.

12. Procédé selon l'une quelconque des revendications 1 à 3 et 10, caractérisé par le fait que le pH de la composition (B) est compris entre 7 et 12 et de préférence entre 8 et 11.

13. Procédé selon l'une quelconque des revendications 1 à 12, caractérisé par le fait que le colorant indolique de formule (I) est présent dans la composition (A) dans des proportions comprises entre 0,1 et 5% en poids et de préférence entre 0,2 et 2% en poids par rapport au poids total de la composition.

14. Procédé selon l'une quelconque des revendications 1 à 13 et 5 à 9, caractérisé par le fait que les précurseurs de colorants d'oxydation para et/ou les colorants d'oxydation "rapides" sont présents dans la composition (B) dans des proportions comprises entre 0,05 et 3% et de préférence entre 0,1 et 2% en poids par rapport au poids total de la composition (B).

15. Procédé selon l'une quelconque des revendications 1 à 3, 5 à 10, 12 à 14, caractérisé par le fait que la composition (B) contient en outre un coupleur dans des proportions n'excédant pas 3% en poids par rapport au poids total de la composition.

16. Procédé selon l'une quelconque des revendications 1 à 15, caractérisé par le fait que le milieu aqueux contient jusqu'à 30% de solvant choisi parmi les alcools, les glycols, les éthers de glycol, le lactate de méthyle.

17. Procédé selon l'une quelconque des revendications 1 à 16, caractérisé par le fait que les compositions (A) et/ou (B) se présentent sous forme de lotion épaissie ou non, de gel ou de mousse aérosol.

18. Procédé selon l'une quelconque des revendications 1 à 17, caractérisé par le fait que la composition (A) appliquée dans un premier temps est maintenue au contact des cheveux pendant une durée comprise entre 1 minute et 60 minutes.

**19.** Procédé selon l'une quelconque des revendications 1 à 18, caractérisé par le fait que la composition (B) est appliquée dans le second temps sur les fibres traitées avec la composition (A), soit immédiatement après l'application de cette dernière, soit de façon décalée dans un laps de temps pouvant varier entre quelques minutes et plusieurs jours.

**20.** Procédé selon la revendication 19, caractérisé par le fait que la composition (B) est maintenue au contact des cheveux pendant une durée comprise entre 1 minute et 60 minutes.

**21.** Agent de teinture des cheveux, caractérisé par le fait qu'il comprend deux composants, le premier composant étant constitué par la composition (A) telle que définie dans l'une quelconque des revendications 1 à 4,10,11,13,16,17, et le second composant étant constitué par une composition (B) telle que définie dans l'une quelconque des revendications 1 à 3, 5 à 10, 12, 14 à 17.

**22.** Dispositif à plusieurs compartiments ou kit de teinture, caractérisé par le fait qu'il comprend dans un premier compartiment, une composition (A) telle que définie dans l'une quelconque des revendications 1 à 4,10,11,13,16,17 et dans un second compartiment, une composition (B) telle que définie dans l'une quelconque des revendications 1 à 3, 5 à 10, 12, 14 à 17.

## Patentansprüche

**1.** Färbeverfahren für keratinische Fasern, insbesondere für menschliche keratinische Fasern, dadurch gekennzeichnet, daß man appliziert:
- in einer ersten Stufe eine Zusammensetzung (A), die in einem zur Färbung geeigneten Milieu, das frei von Oxidationsmitteln und Oxidationskatalysatoren ist, mindestens einen Indolfarbstoff der Formel enthält:

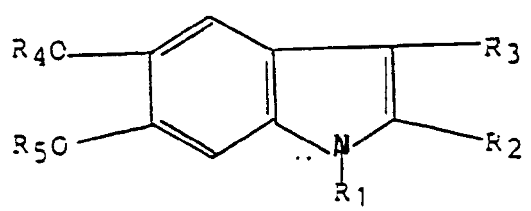

(I)

= worin bedeuten: $R_1$ ein Wasserstoffatom, eine Niederalkylgruppe oder eine -$SiR_6R_7R_8$-Gruppe;

$R_2$ und $R_3$, die gleich oder verschieden sind, ein Wasserstoffatom oder auch eine Niederalkylgruppe, eine Carboxylgruppe, eine Niederalkoxy-carbonylgruppe oder eine -$COOSiR_8R_7R_8$-Gruppe bedeuten;

$R_4$ und $R_8$ die gleich oder verschieden sind, bedeuten ein Wasserstoffatom, eine geradkettige oder verzweigte $C_1$-$C_{20}$-Alkylgruppe, eine Formylgruppe, eine geradkettige oder verzweifle $C_2$-$C_{20}$-Acylgruppe, eine geradkettige oder verzweigte $C_3$-$C_{20}$-Alkenoylgruppe, eine -$SiR_8R_7R_8$-Gruppe, eine -$P(O)(OR_9)_2$-Gruppe, eine -$SO_2OR_9$-Gruppe, oder $R_4$ und $R_8$ zusammen mit den Sauerstoffatomen, mit denen sie verbunden sind, auch einen Ring bilden, der gegebenenfalls eine Carbonylgruppe, eine Methylengruppe, eine Thiocarbonylgruppe oder eine Gruppe enthält:

$>P(O)OR_9$, oder auch $>CR_{10}R_{11}$;

$R_9$ und $R_{10}$ bedeuten ein Wasserstoffatom oder eine Niederalkylgruppe, $R_{11}$ eine Niederalkoxygruppe oder eine Mono- oder Diniederalkylaminogruppe, $R_6$, $R_7$ und $R_8$, die gleich oder verschieden sind, bedeuten geradkettige oder verzweigte Niederalkylgruppen;

und die Säureadditionssalze mit anorganischen oder anorganischen Säuren, wie z.B. die entsprechenden Alkalimetall-, Erdalkalimetall- oder Aminsalze;

- in einer zweiten Stufe appliziert man dann auf diese Fasern eine Zusammensetzung (B), die in einem zur Färbung geeigneten Milieu, das frei ist von Oxidationskatalysatoren, mindestens einen Oxidationsfarbstoff-Vorläufer vom para-Typ enthält, ausgewählt aus Paraphenylendiaminen, Paraaminophenolen, und Para-heterocyclischen Basen, ausgewählt aus den Pyridin- oder Pyrimidinderivaten, und/oder mindestens einen "schnellen" Oxidationsfarbstoff, ausgewählt aus den Trihydroxylderivaten von Benzol, den Diaminohydroxybenzolen, den Aminodihydroxybenzolen, den substituierten Aminohydroxybenzolen, den Triaminobenzolen, den substituierten 1,2-Dihydroxybenzolen, Brasilin, Hämatoxylin und Alkanna-Extrakt, wobei die Zusammensetzung (B) gegebenenfalls zum Zeitpunkt der Anwendung mit einer Wasserstoffperoxid-Lösung gemischt

= wird.

2. Verfahren nach Anspruch 1, dadurch gekennzeichnet, daß man die Fasern nach Applikation der Zusammensetzung (A) spült.

3. Verfahren nach Anspruch 2, dadurch gekennzeichnet, daß man nach der Spülung vor der Applikation der Zusammensetzung (B) eine Trocknung der Haare durchführt.

4. Verfahren nach einem der Ansprüche 1 bis 3, dadurch gekennzeichnet, daß die Indolfarbstoffe der Formel (I) ausgewählt sind aus 5,6-Dihydroxyindol, 2-Methyl-5,6-dihydroxyindol, 5-Methoxy-6-hydroxy-indol, (5 oder 6)-Acetoxy-(6 oder 5)-hydroxy-indol, 2-Carboxy-5,6-dihydroxy-indol, 3-Methyl-5,6-dihydroxy-indol, 2,3-Dimethyl-5,6-dihydroxy-indol, und ihren Salzen.

5. Verfahren nach einem der Ansprüche 1 bis 3, dadurch gekennzeichnet, daß die Paraphenylendiamine des Formel (II) entsprechen:

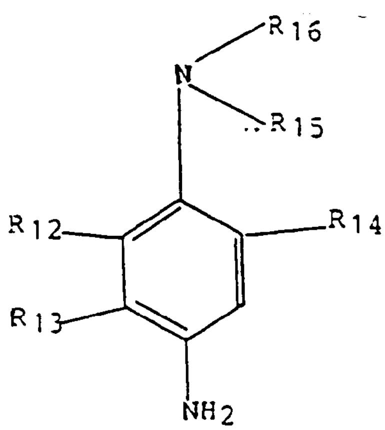

(II)

worin bedeuten:

$R_{12}$, $R_{13}$, $R_{14}$, die gleich oder verschieden sind, ein Wasserstoffatom oder Halogenatom, ein Niederalkylradikal, ein Niederalkoxyradikal;

$R_{15}$ und $R_{16}$, die gleich oder verschieden sind, bedeuten ein Wasserstoffatom, eine Niederalkylgruppe, eine Hydroxy-Niederalkylgruppe, eine Niederalkoxyalkylgruppe, eine Carbamoyl-Niederalkylgruppe, eine Sulfo-Niederalkylgruppe, eine Mesylamino-Niederalkylgruppe, eine Acetylamino-Niederalkylgruppe, eine Ureido-Niederalkylgruppe, eine Carbetoxyamino-Niederalkylgruppe, eine Piperidino-Niederalkylgruppe, eine Morpholino-Niederalkylgruppe, oder $R_{15}$ und $R_{16}$ können zusammen mit dem Stickstoffatom, an das sie gebunden sind, einen Piperidin- oder Morpholin-Heterocyclus bilden, mit der Maßgabe, daß $R_{12}$ und $R_{14}$ ein Wasserstoffatom bedeuten, wenn $R_{15}$ und $R_{16}$ kein Wasserstoffatom bedeuten;

sowie die entsprechenden Salze.

6. Verfahren nach einem der Ansprüche 1 bis 3 und 5, dadurch gekennzeichnet, daß die Paraphenylendiamine ausgewählt sind unter p-Phenylendiamin, p-Toluylendiamin, Methoxy-paraphenylendiamin, Chlorparaphenylendiamin, 2,6-Dimethyl-p-phenylendiamin, 2,5-Dimethyl-paraphenylendiamin, 2-Methyl-5-methoxy-paraphenylendiamin, 2,6-Dimethyl-5-methoxy-paraphenylendiamin, N,N-Dimethylparaphenylendiamin, 3-Methyl-4-amino-N,N-diethyl-anilin, N,N-Di-(β-Hydroxyethyl)-paraphenylendiamin, 3-Methyl-4-amino-N,N-di-(β-hydroxyethyl)-anilin, 3-Chlor-4-amino-N,N-di-(β-hydroxyethyl)-anilin, 4-Amino-N,N-(ethylcarbamoylmethyl)-anilin, 3-Methyl-4-amino-N,N-(ethyl-carbamoylmethyl)-anilin, 4-Amino-N,N-(ethyl-β-piperidinoethyl)-anilin, 3-Methyl-4-amino-N,N-(ethyl-β-piperidinoethyl)-anilin, 4-Amino-N,N-(ethyl-β-morpholinoethyl)-anilin, 3-Methyl-4-amino-N,N-(ethyl-β-morpholinoethyl)-anilin, 4-Amino-N,N-(ethyl-β-acetylaminoethyl)-anilin, 4-Amino-N-β-methoxyethylanilin, 3-Methyl-4-amino-N,N-(ethyl-β-acethylaminoethyl)-anilin, 4-Amino-N,N-(ethyl-β-mesylaminoethyl)-anilin, 3-Methyl-4-amino-N,N-(ethyl-β-mesylaminoethyl)-anilin, 4-Amino-N,N-(ethyl-β-sulfoethyl)-anilin, 3-Methyl-4-amino-N,N-(ethyl-β-sulfoethyl)-anilin, N-(4-Aminophenyl)-morpholin, N-(4-Aminophenyl)-piperidin, 2,3,-Dimethyl-p-phenylendiamin, Isopropyl-p-phenylendiamin, und ihren Salzen.

7. Verfahren nach einem der Ansprüche 1 bis 3, dadurch gekennzeichnet, daß die Paraaminophenole ausgewählt sind aus Paraaminophenol, N-Methyl-p-aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol.

8. Verfahren nach einem der Ansprüche 1 bis 3, dadurch gekennzeichnet, daß die para-heterocyclischen Oxidationsfarbstoffe ausgewählt sind unter 2,5-Diaminopyridin, 2-Hydroxy-5-aminopyridin und Tetraaminopyrimidin.

9. Verfahren nach einem der Ansprüche 1 bis 3, dadurch gekennzeichnet, daß die "schnellen" Oxidationsfarbstoffe ausgewählt sind unter 1,2,4-Trihydroxy-benzol, 1,2,4-Trihydroxy-5-($C_1$-$C_6$)-alkylbenzol, 4-Amino-resorcin, 1,2,3-Trihydroxy-benzol, 4-Methyl-1,2-dihydroxy-benzol, 2-Amino-1,4-dihydroxy-benzol, 2-Amino-4-methoxyphenol, 2,4-Diamino-phenol, 3-Methoxy-1,2-dihydroxy-benzol, 1,4-Dihydroxy-2-N,N-diethylamino-benzol, 2,5-Diamino-4-methoxy-1-hydroxy-benzol, 4,6-Dimethoxy-3-amino-1-hydroxy-benzol, 2,6-Dimethyl-4-N-p-hydroxyphenylamino-1-hydroxy-benzol, 1,5-Diamino-2-methyl-4-N-p-hydroxyphenylamino-benzol, und ihren Salzen.

10. Verfahren nach einem der Ansprüche 1 bis 9, dadurch gekennzeichnet, daß das zur Färbung geeignete Milieu ein wässeriges Milieu ist, das aus Wasser oder einer Mischung Wasser/Lösungsmittel besteht.

11. Verfahren nach einem der Ansprüche 1 bis 4 und 10, dadurch gekennzeichnet, daß der pH-Wert der Zusammensetzung (A) zwischen 4 und 11, und vorzugsweise zwischen 6 und 10 liegt.

12. Verfahren nach einem der Ansprüche 1 bis 3 und 10, dadurch gekennzeichnet, daß der pH-Wert der Zusammensetzung (B) zwischen 7 und 12, und vorzugsweise zwischen 8 und 11 liegt.

13. Verfahren nach einem der Ansprüche 1 bis 12, dadurch gekennzeichnet, daß der Indolfarbstoff der Formel (I) in der Zusammensetzung (A) in Anteilen zwischen 0.1 und 5 Gew.-%, und vorzugsweise zwischen 0.2 und 2 Gew.-%, bezogen auf die gesamte Zusammensetzung, vorhanden ist.

14. Verfahren nach einem der Ansprüche 1 bis 13 und 5 bis 9, dadurch gekennzeichnet, daß die Oxidationsfarbstoff-Vorläufer vom Para-Typ und/oder die "schnellen" Oxidationsfarbstoffe in der Zusammensetzung (B) in Anteilen zwischen 0.05 bis 3, vorzugsweise zwischen 0.1 und 2 Gew.-%, bezogen auf die gesamte Zusammensetzung (B), vorhanden sind.

15. Verfahren nach einem der Ansprüche 1 bis 3, 5 bis 10, 12 bis 14, dadurch gekennzeichnet, daß die Zusammensetzung (B) außerdem einen Kuppler in Anteilen enthält, die 3 Gew.-%, bezogen auf die gesamte Zusammensetzung, nicht überschreiten.

16. Verfahren nach einem der Ansprüche 1 bis 15, dadurch gekennzeichnet, daß das wässerige Milieu bis zu 30% eines Lösungsmittels, ausgewählt unter Alkoholen, Glycolen, Glycolethern, Methyllactat, enthält.

17. Verfahren nach einem der Ansprüche 1 bis 16, dadurch gekennzeichnet, daß die Zusammensetzungen (A) und/oder (B) in Form einer verdickten oder nicht verdickten Lotion, eines Gels oder eines Aerosolschaumes vorliegen.

18. Verfahren nach einem der Ansprüche 1 bis 17, dadurch gekennzeichnet, daß die Zusammensetzung (A), die in der erste Stufe appliziert wird, während einer Dauer zwischen 1 Minute und 60 Minuten in Kontakt mit den Haaren verbleibt.

19. Verfahren nach einem der Ansprüche 1 bis 18, dadurch gekennzeichnet, daß die Zusammensetzung (B) in der zweiten Stufe auf die mit der Zusammensetzung (A) behandelten Haare entweder sofort nach der Applikation der letzteren, oder zeitlich verschoben nach einem Zeitraum, der zwischen einigen Minuten und mehreren Stunden variieren kann, appliziert wird.

20. Verfahren nach Anspruch 19, dadurch gekennzeichnet, daß die Zusammensetzung (B) während einer Dauer zwischen 1 Minute und 60 Minuten in Kontakt mit den Haaren verbleibt.

21. Färbemittel für Haare, dadurch gekennzeichnet, daß es zwei Komponenten enthält, wobei die erste Komponente aus der Zusammensetzung (A) besteht, wie sie in einem der Ansprüche 1 bis 4, 10, 11, 13, 16, 17 definiert ist, und die zweite Komponente aus einer Zusammensetzung (B) besteht, wie sie in einem der Ansprüche 1 bis 3, 5 bis 10, 12, 14 bis 17 definiert ist.

**22.** Vorrichtung mit mehreren Kompartimenten oder Färbekit, dadurch gekennzeichnet, daß sie in einem ersten Kompartiment eine Zusammensetzung (A), wie sie in einem der Ansprüche 1 bis 4, 10, 11, 13, 16, 17 definiert ist, enthält, und in einem zweiten Kompartiment eine Zusammensetzung (B), wie sie in einem der Ansprüche 1 bis 3, 5 bis 10, 12, 14 bis 17 definiert ist.

## Claims

**1.** Process for dyeing keratinous fibres, especially human keratinous fibres, characterized in that there is applied on these fibres :

- in a first step, a composition (A) containing, in a medium suitable for dyeing and free from all oxidizing agents and from all oxidation catalyst , at least one indole dye corresponding to the formula :

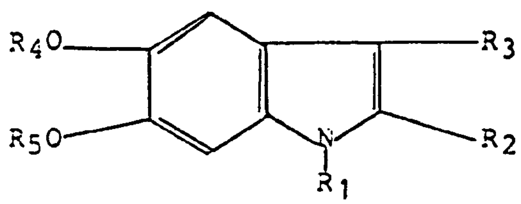

(I)

in which:

$R_1$ represents a hydrogen atom, a lower alkyl group or a group $-SiR_6R_7R_8$;

$R_2$ and $R_3$, which may be identical or different, represent a hydrogen atom or alternatively a lower alkyl group, a carboxyl group, a (lower alkoxy)carbonyl group or a group $-COOSiR_8R_7R_8$;

$R_4$ and $R_8$, which may be identical or different, represent a hydrogen atom, a linear or branched $C_1$-$C_{20}$ alkyl group, a formyl group, a linear or branched $C_2$-$C_{20}$ acyl group, a linear or branched $C_3$-$C_{20}$ alkenoyl group, a group $-SiR_8R_7R_8$, a group $-P(O)(OR_9)_2$, or a group $-SO_2OR_9$, or alternatively $R_4$ and $R_8$, together with the oxygen atoms to which they are attached, form a ring optionally containing a carbonyl group, a methylene group, a thiocarbonyl group or a group:

$>P(O)OR_9$, or alternatively $>CR_{10}R_{11}$; and

$R_9$- and $R_{10}$ represent a hydrogen atom or a lower alkyl group, $R_{11}$ representing a lower alkoxy group or a mono- or dialkylamino group, $R_8$, $R_7$ and $R_8$, which may be identical or different, representing linear or branched lower alkyl groups;

and the addition salts with inorganic or organic acids as well as the corresponding alkali metal, alkaline earth metal or amine salts;

and

- in a second step, there is applied on these fibres a composition (B) containing, in a medium suitable for dyeing and free from all oxidation catalyst, at least one para oxidation dye precursor selected from paraphenylenediamines, para-aminophenols and heterocyclic para bases selected from derivatives of pyridine or of pyrimidine, and/or at least one so-called "rapid" oxidation dye selected from trihydroxylated derivatives of benzene , diaminohydroxybenzenes,aminodihydroxybenzenes, substituted aminohydroxybenzenes, triaminobenzenes, substituted 1,2-dihydroxybenzenes,brazilin, haematoxylin and alkanet extract, the composition (B) optionally being mixed at the time of use with a hydrogen peroxide solution.

**2.** Process according to Claim 1, characterized in that a rinsing of the treated fibres is performed after application of the composition (A).

**3.** Process according to Claim 2, characterized in that this rinsing is followed by a step of drying the hair before the application of the composition (B).

**4.** Process according to any one of Claims 1 to 3, characterized in that the indole dyes of the formula (I) are selected from 5,6-dihydroxyindole, 2-methyl-5,6-dihydroxyindole, 5-methoxy-6-hydroxyindole, (5 or 6)-acetoxy-(6 or 5)-hydroxyindole, 2-carboxy-5,6-dihydroxyindole,3-methyl-5,6-dihydroxyindole, 2,3-dimethyl-5,6-dihydroxyindole and their salts.

5. Process according to any one of Claims 1 to 3, characterized in that the para-phenylenediamines correspond to the formula (II) :

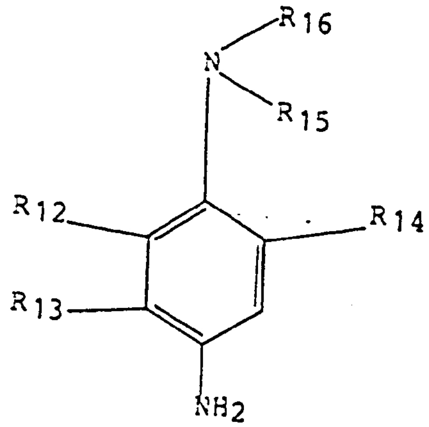

(II)

in which:

- $R_{12}$, $R_{13}$, $R_{14}$, which may be identical or different, denote a hydrogen or halogen atom, a lower alkyl radical or a lower alkoxy radical;
- $R_{15}$ and $R_{16}$, which may be identical or different, denote a hydrogen atom or a lower alkyl, lower hydroxyalkyl, lower alkoxyalkyl, lower carbamylalkyl, lower sulphoalkyl, lower mesylaminoalkyl, lower acetylaminoalkyl, lower ureidoalkyl, lower carbethoxyaminoalkyl, lower piperidinoalkyl or lower morpholinoalkyl group, or alternatively $R_{15}$ and $R_{16}$, together with the atom to which they are attached, form a piperidino or morpholino heterocyle, with the proviso that $R_{12}$ and $R_{14}$ represent a hydrogen atom when $R_{15}$ and $R_{16}$ do not represent a hydrogen atom; as well the corresponding salts.

6. Process according to any one of Claims 1 to 3 and 5, characterized in that the para-phenylenediamines are selected from the p-phenylenediamine, p-toluenediamine, methoxy-para-phenylenediamine, chloro-para-phenylenediamine, 2,6-dimethyl-p-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-bis(β-hydroxyethyl)-para-phenylenediamine, 3-methyl-4-amino-N,N-bis(β-hydroxyethyl)aniline, 3-chloro-4-amino-N,N-bis(β-hydroxyethyl)aniline, 4-amino-N-ethyl-N-(carbamylmethyl)aniline, 3-methyl-4-amino-N-ethyl-N-(carbamylmethyl)aniline, 4-amino-N-ethyl-N-(β-piperidinoethyl)aniline, 3-methyl-4-amino-N-ethyl-N-(β-piperidinoethyl)aniline, 4-amino-N-ethyl-N-(β-morpholinoethyl)aniline, 3-methyl-4-amino-N-ethyl-N-(β-morpholinoethyl)aniline, 4-amino-N-ethyl-N-(β-acetylaminoethyl)-aniline, 4-amino-N-(β-methoxyethyl)aniline, 3-methyl-4-amino-N-ethyl-N-(β-acetylaminoethyl)- aniline, 4-amino-N-ethyl-N-(β-mesylaminoethyl)aniline, 3-methyl-4-amino-N-ethyl-N-(β-mesylaminoethyl)aniline, 4-amino-N-ethyl-N-(β-sulphoethyl)aniline, 3-methyl-4-amino-N-ethyl-N-(β-sulphoethyl)aniline, N-(4-aminophenyl)morpholine, N-(4-aminophenyl)piperidine, 2,3-dimethyl-p-phenylenediamine, isopropyl-p-phenylenediamine and their salts.

7. Process according to any one of Claims 1 to 3, characterized in that the para-aminophenols are selected from para-aminophenol, N-methyl-p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

8. Process according to any one of Claims 1 to 3, characterized in that the heterocyclic para oxidation dyes are selected from 2,5-diaminopyridine, 2-hydroxy-5-aminopyridine and tetraaminopyrimidine.

9. Process according to any one of Claims 1 to 3, characterized in that the "rapid" oxidation dye is selected from 1,2,4-trihydroxybenzene, 1,2,4-trihydroxy-5-($C_1$-$C_6$ alkyl)benzene, 4-aminoresorcinol, 1,2,3-trihydroxybenzene, 4-methyl-1,2-dihydroxybenzene, 2-amino-1,4-dihydroxybenzene, 2-amino-4-methoxyphenol,2,4-diaminophenol,3-methoxy-1,2-dihydroxybenzene, 1,4-dihydroxy-2-(N,N-diethylamino)benzene,2,5-diamino-4-methoxy-1-hydroxybenzene, 4,6-dimethoxy-3-amino-1-hydroxybenzene, 2,6-dimethyl-4- [N-(p-hydroxyphenyl)amino]-1-hydroxybenzene, 1,5-diamino-2-methyl-4-[N-(p-hydroxyphenyl)-amino]benzene and their salts.

**10.** Process according to any one of Claims 1 to 9, characterized in that the medium suitable for dyeing is an aqueous medium consisting of water or a water/solvent(s) mixture.

**11.** Process according to any one of Claims 1 to 4 and 10, characterized in that the pH of the composition (A) is between 4 and 11, and preferably between 6 and 10.

**12.** Process according to any one of Claims 1 to 3 and 10, characterized in that the pH of the composition (B) is betweeen 7 and 12, and preferably between 8 and 11.

**13.** Process according to any one of Claims 1 to 12, characterized in that the indole dye of formula (I) is present in the composition (A) in proportions of between 0.1 and 5% by weight, and preferably between 0.2 and 2% by weight, relative to the total weight of the composition.

**14.** Process according to any one of Claims 1 to 13 and 5 to 9 characterized in that the para-oxidation dye precursors and/or the "rapid" oxidation dyes are present in the composition (B) in proportions of between 0.05 and 3% and preferably between 0.1 and 2% by weight relative to the total weight of the composition (B).

**15.** Process according to any one of claims 1 to 3, 5 to 10 and 12 to 14, characterized in that the composition (B) contains, in addition, a coupler in proportions not exceeding 3% by weight relative to the total weight of the composition.

**16.** Process according to any one of Claims 1 to 15, characterized in that the aqueous medium contains up to 30% of solvent selected from alcohols, glycols, glycol ethers and methyl lactate.

**17.** Process according to any one of Claims 1 to 16,characterized in that the compositions (A) and/or (B) are presented in the form of a lotion, thickened or otherwise, a gel or an aerosol mousse.

**18.** Process according to any one of Claims 1 to 17, characterized in that the composition (A) applied in a first stage is maintained in contact with the hair for a period of between 1 minute and 60 minutes.

**19.** Process according to any one of Claims 1 to 18, characterized in that the composition (B) is applied in a second stage on the fibres treated with the composition (A), either immediately after the application of the latter, or postponing the second stage for a lapse of time which can vary between a few minutes and several days.

**20.** Process according to Claim 19, characterized in that the composition (B) is maintained in contact with the hair for a period of between 1 minute and 60 minutes.

**21.** Hair dyeing agent, characterized in that it comprises two components, the first component consisting of the composition (A) as defined in any one of Claims 1 to 4,10,11,13,16 and 17, and the second component consisting of a composition (B) as defined in any one of Claims 1 to 3, 5 to 10 and 14 to 17.

**22.** Multi-compartment device or dyeing kit, characterized in that it comprises a composition (A) as defined in any one of Claims 1 to 4, 10,11,13,16 and 17 in a first compartment, and a composition (B) as defined in any one of Claims 1 to 3, 5 to 10,12 and 14 to 17 in second compartment.